# EUROPEAN PATENT SPECIFICATION

(11) **EP 1 475 558 B1**
(45) Date of publication and mention of the grant of the patent: **27.09.2006**
(21) Application number: 04380100.0
(22) Date of filing: 03.05.2004
(51) Int. Cl.: F16K 31/04

(54) **A liquid distributor valve**
Flüssigkeitverteilerventil
Vanne de distribution de fluides

(30) Priority: 09.05.2003 ES 200301127
(43) Date of publication of application: 10.11.2004
(73) Proprietor: Valvules i Racords Canovelles, S.A., 08150 Parets del Valles (Barcelona) (ES)
(72) Inventor: Ibanez Sapina, Miguel c/o Valv. y Racords Canovell, 08150 Parets del Valles (Barcelona) (ES); Puiggros Roig, Armand c/o Valv. y Racords Canovell, 08150 Parets del Valles (Barcelona) (ES)
(74) Representative: Pastells Teixido, Manuel

(56) References cited:
- EP-A- 0 287 457
- EP-A- 1 150 050
- AU-B- 500 746
- DE-U- 9 010 608
- GB-A- 886 632
- US-A- 3 870 274

## Description

### Background of the invention

Liquid distributor valves are known which comprise a radially compartmented base body having several outlets and an upperly arranged, lateral inlet, a rotatable obturator bell being interposed between said outlets and said inlet and being provided with an upperly arranged shaft projecting through the top cover of said body and being actuated by a manual or electronic, externally arranged control.

This type of electronically actuated valves has the drawback that the electronic control portion and the mechanical portion acting within the base body are linked to each other thus forming an assembly. This represents a substantial drawback when there is a need to repair the electronic portion or the mechanical portion, since the assembly being made up by them must then be disassembled in order to replace or repair the affected components.

This very applicant is the owner of the EP-1 150 050 having as its object a liquid distributor of the aforementioned type wherein the electronic control forms an independent assembly being fit to be removably mounted onto the base body, the obturator bell having a shaft that penetrates into the electronic control and is coupled to the shaft of the gearmotor being comprised in said electronic control, the object of this patent being that of providing the distributor with means allowing to simplify the actuation being exerted on the obturator bell thus producing its rotation with no need to lift and separate it with respect to the opening of the compartments, the bell being hence directly rotated in a sliding motion on said openings.

Documents EP-A-0 287 457 and DE 9010608 disclose further prior art liquid distributors.

### Summary of the invention

This invention has as its object the provision of means allowing coupling the obturator bell shaft to the shaft of the gearmotor belonging to the electronic control. These means are characterised in that they comprise a cap being fitted onto the bell shaft and being provided with a screw being inserted into its top opening, said screw threadingly engaging an axial thread of said shaft and comprising a head being provided with recesses being apt to engagingly receive protrusions of the gearmotor shaft.

The outer periphery of the above-mentioned cap forms a dentation in such a way that together and in cooperation with a resiliently biased pawl being provided in the base serving as a support for the electronic, externally arranged control said dentation makes up a ratchet allowing to stabilise the different rotational positions of the obturator bell, said cap being radially provided with an appendage being apt to act on the microswitches equipping the electronic circuit of the control in order to thus determine said positions of the obturator bell.

The screw being fitted onto the obturator bell shaft has a flange abuttingly contacting a limit switch being provided at the gearmotor in order to thus limit the lifting motion of said screw and bell.

These and other characteristic features will be best made apparent by the following detailed description whose understanding will be made easier by the accompanying two sheets of drawings showing a practical embodiment being cited only by way of example not limiting the scope of the present invention.

### Description of the drawings

In the drawings:

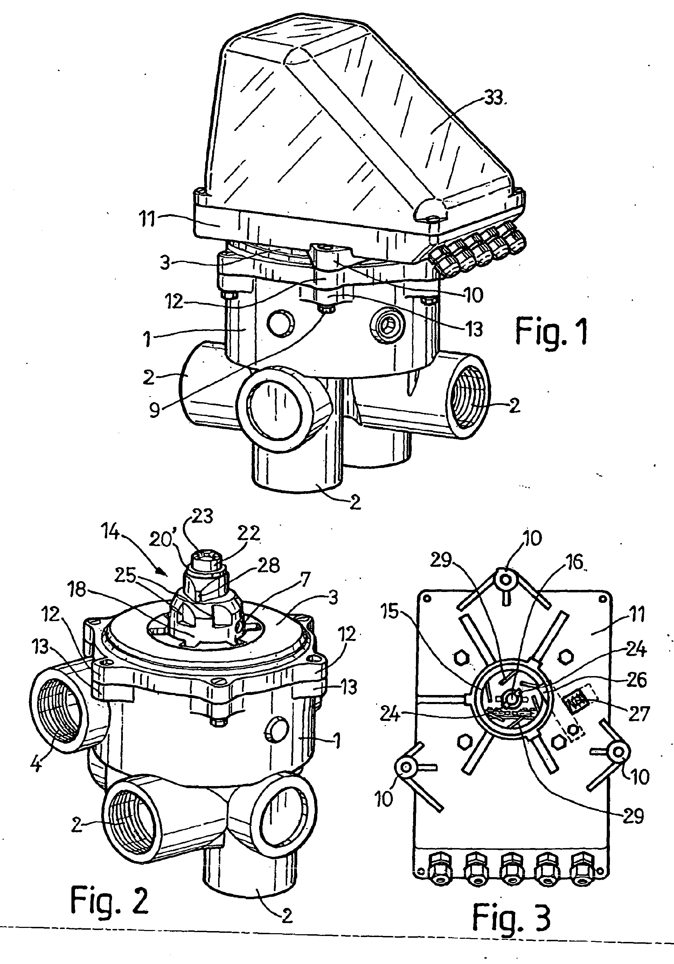

Fig. 1 illustrates in a perspective view the distributor valve being the object of the invention;
Fig. 2 shows in a perspective view the base body of said valve;
Fig. 3 represents in a bottom plan-view the assembly making up the electronic, externally arranged control;
Fig. 4 is a perspective view showing in detail the means allowing to couple the obturator bell shaft to the gearmotor shaft of the electronic control; and
Figs. 5 and 6 are each a perspective view respectively showing in one of them the microswitches and the gearmotor, and in the other one the gearmotor shaft and the limit switch.

### Detailed description

According to the drawings this liquid distributor valve comprises a compartmented base body (1) having several outlets (2) and a lateral inlet (4), a cover (3) being fastened onto said base body.

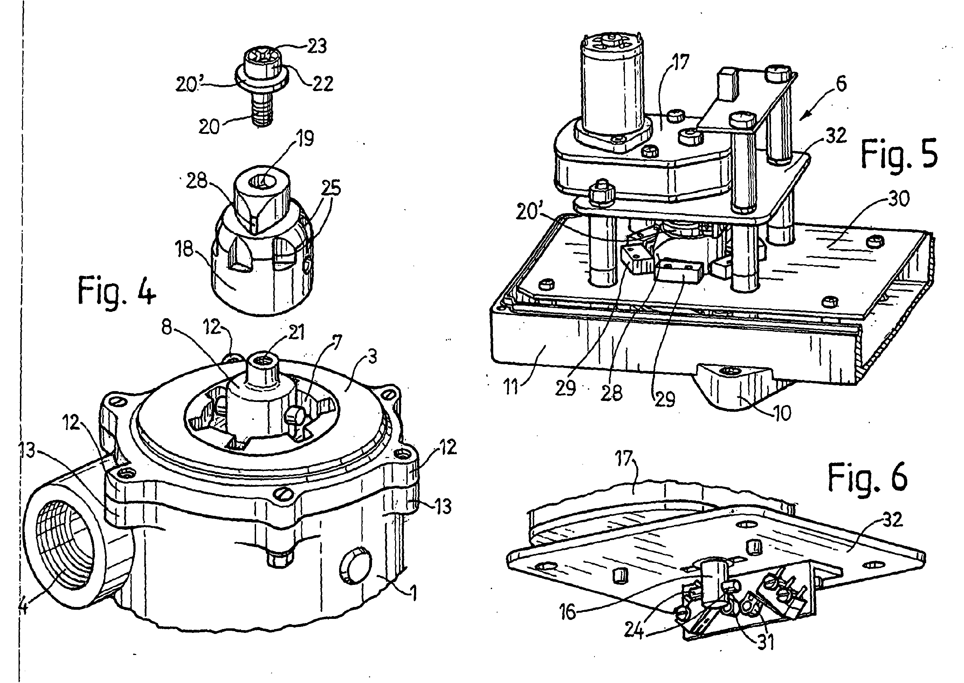

The base body (1) does innerly comprise a rotatable obturator bell being arranged between the outlets (2) and the inlet (4), said bell being governed by an electronic, externally arranged control (6) being superimposed on the cover (3), a shaft (8) corresponding to the obturator bell projecting through a central orifice (7) of said cover (Fig. 4).

The externally arranged control (6) forms an independent assembly being apt to be removably fitted onto the base body (1). In this embodiment this fitting is carried out by means of three bolts extending each through a respective eye lug (10) being provided at the contour of the base (11) serving as a support for the electronic, externally arranged control (6), through a respective eye lug (12) being provided at the periphery of the cover (3), and through a respective eye lug (13) being provided at the periphery of the base body (1), said bolts being threadingly engaged by the corresponding nuts (9). Said fitting of the electronic, externally arranged control (6) to the base body (1) can also be carried out through other means, such as by means of a bayonet attachment.

The obturator bell shaft (8) comprises coupling means (14) being apt to penetrate into the assembly making up the electronic, externally arranged control (6) through an orifice (15) of the base (11) to thus be coupled to the shaft (16) of the gearmotor (17) pertaining to said control (6).

These coupling means (14) comprise a cap (18) being apt to be fitted onto the obturator bell shaft (8), a screw (20) being inserted into the top opening (19) of said cap and being thus threadingly engaged with an axial thread (21) being provided in said shaft, said screw comprising a head (22) having recesses (23) being arranged in a crosswise arrangement and being apt to engagingly receive two opposite protrusions (24) of the gearmotor shaft (16).

The outer periphery of cap (18) forms a dentation (25) in such a way that together and in cooperation with a pawl (26) being resiliently biased by a spring (27), both of them being provided in the base (11), said dentation makes up a ratchet allowing to stabilise the different rotational positions of the obturator bell, said cap (8) being radially provided with an appendage (28) being apt to act on the microswitches (29) equipping the electronic circuit being printed on the board (30) and comprising all of the components (not shown) integrating the control (6) in order to thus determine said positions of the obturator bell.

The screw (20) has a flange (20') abuttingly contacting a limit switch (31) being provided at the gearmotor (17) in order to thus limit the lifting motion of said screw and obturator bell, said gearmotor being supported by a plate (32) being installed above the printed circuit board (30).

Whenever wishing to manually actuate this valve, upon removal of cap (18) and screw (20) the shaft (8) and the cover (3) are prepared to receive the coupling of a handle.

The electronic, externally arranged control (6) is protected by means of a cover (33) being apt to be removably fitted onto the base (11).

The invention can within its essentiality be put into practice in other embodiments only in detail differing from the one having been described above only by way of example, said other embodiments also falling within the scope of the protection being sought.

## Claims

1. A liquid distributor valve comprising a compartmented base body (1) being provided with a top cover (3) and having several outlets (2) and an upperly arranged, lateral inlet (4), a rotatable obturator bell being arranged between said outlets and said inlet and having an upperly arranged shaft (8) projecting through said cover (3), said shaft being governed by an electronic, externally arranged control (6), the electronic, externally arranged control forming an independent assembly being apt to be removably fitted onto the base body (1), the obturator bell shaft (8) comprising means (14) being apt to allow to removably couple it, said means also being apt to penetrate into the assembly forming the electronic, externally arranged control (6) and to be coupled to the shaft (16) of the gearmotor (17) pertaining to said electronic, externally arranged control (6); **characterised in that** the means allowing to couple the obturator bell shaft (8) to the shaft (16) of the gearmotor (17) comprise a cap (18) being fitted onto the obturator bell shaft (8) and being provided with a screw (20) being inserted into its top opening (19), said screw threadingly engaging an axial thread (21) of said shaft (8) and comprising a head (22) being provided with recesses (23) being apt to engagingly receive protrusions (24) of the shaft (16) of the gearmotor (17).

2. A liquid distributor valve as per claim 1, **characterised in that** the outer periphery of the cap (18) forms a dentation (25) in such a way that together and in cooperation with a resiliently biased pawl (26) being provided in the base (11) serving as a support for the electronic, externally arranged control (6) said dentation makes up a ratchet allowing to stabilise the different rotational positions of the obturator bell, said cap (8) being radially provided with an appendage (28) being apt to act on the microswitches (29) equipping the electronic circuit (30) of the control (6) in order to thus determine said positions of the obturator bell.

3. A liquid distributor valve as per claim 1, **characterised in that** the screw (20) being fitted onto the obturator bell shaft (8) has a flange (20') abuttingly contacting a limit switch being provided at the gearmotor (17) in order to thus limit the lifting motion of said screw (20) and bell.

4. A liquid distributor valve as per claim 1, **characterised in that** upon removal of said cap (18) and screw (20) the obturator bell shaft (8) is prepared to receive the coupling of a handle allowing to manually actuate said bell.

## Patentansprüche

1. Flüssigkeitsverteilerventil, das einen innerlich durch Trennwände geteilten Grundkörper (1) umfaßt, der mit einer oberen Abdeckung (3) versehen ist und verschiedene Auslässe (2) sowie einen oben angeordneten, seitlichen Einlaß (4) hat, wobei eine drehbare Absperrglocke zwischen den obengenannten Auslässen und dem obengenannten Einlaß angeordnet ist und eine oben angeordnete Spindel (8) hat, die durch die obengenannte Abdeckung (3) hindurchragt, wobei die obengenannte Spindel von einer elektronischen, äußerlich angeordneten Steuerung gesteuert wird, wobei die elektronische, äußerlich angeordnete Steuerung eine unabhängige, in lösbarer Weise auf dem Grundkörper (1) anbringbare Einheit bildet, wobei die Absperrglockenspindel (8) Mittel (14) unfaßt, die ihre lösbare Kopplung erlauben, wobei die obengenannten Mittel auch in die die elektronische, äußerlich angeordnte Steuerung (6) bildende Einheit einführbar und zur Welle (16) des zur obengenannten elektronischen, äußerlich angeordneten Steuerung (6) gehörenden Getriebemotors (17) koppelbar sind; **dadurch gekennzeichnet, daß** die die Kopplung der Absperrglockenspindel (8) zur Welle (16) des Getriebemotors (17) erlaubenden Mittel eine auf der Absperrglockenspindel (8) anbringbare Buchse (18) umfassen, die mit einer in ihre obere Öffnung (19) einführbaren Schraube (20) bestückt ist, wobei die obengenannte Schraube in ein sich in der Achsenrichtung erstreckendes Gewinde (21) der vorgenannten Spindel (8) einschraubbar ist und einen Kopf (22) umfaßt, der mit Aushöhlungen (23) zur gegenseitig anpassenden Aufnahme von Vorsprüngen (24) der Welle (16) des Getriebmotors (17) versehen ist.

2. Flüssigkeitsverteilerventil nach Anspruch 1, **dadurch gekennzeichnet, daß** der äußere Umfang der Buchse (18) eine Verzhanung (25) bildet, in solcher Weise, daß zusammen und in Zusammenarbeit mit einem in der als Halterung für die elektronische, äußerlich angeordnete Steuerung (6) dienenden Auflage (11) angeordneten, federbelasteten Rasthebel (26) die vorgenannte Verzahnung eine Sperrklinke bildet, die es erlaubt, die verschiedenen Drehstellungen der Absperrglocke zu stabilisieren, wobei die vorgenannte Buchse (18) zur entsprechenden Feststellung der vorgenannten Absperrglockenstellungen mit einer sich in radialer Richtung erstreckenden, die die elektronische Schaltung (30) bestückenden Mikroschalter (29) betätigenden Nase (28) versehen ist.

3. Flüssigkeitsverteilerventil nach Anspruch 1, **dadurch gekennzeichnet, daß** die auf der Absperrglockenspindel (8) angebrachte Schraube (20) einen Bund (20') hat, der zur entsprechenden Beschränkung der Hubbewegung der vorgenannten Schraube (20) und Glocke gegen einen auf dem Getriebemotor (17) angeordneten Endschalter anschlagt.

4. Flüssigkeitsverteilerventil nach Anspruch 1, **dadurch gekennzeichnet, daß** nach der Entfernung der vorgenannten Buchse (18) und Schraube (20) die Absperrglockenspindel (8) zur Aufnahme eines auf ihr anbringbaren, zur Handbetätigung der vorgenannten Glocke dienenden Betätigungshebels bereit ist.

## Revendications

1. Valve distributrice pour liquides comprenant un corps de base (1) compartimenté muni d'un couvercle supérieur (3) et ayant plusieurs sorties (2) et une entrée latérale (4) supérieure, une cloche obturante rotative étant disposée entre lesdites sorties et ladite entrée, ladite cloche ayant un axe supérieur (8) sortant à travers dudit couvercle (3) et étant gouverné par une commande électronique extérieure (6), la commande électronique extérieure (6) formant un ensemble indépendant apte à être amoviblement accouplé sur le corps de base (1), l'axe (8) de la cloche obturante comprenant des moyens (14) permettant de l'accoupler amoviblement, lesdits moyens étant aptes aussi à pénétrer dans l'ensemble qui forme la commande électronique extérieure (6) et à être accouplés sur l'axe (16) du moteur à engrenage (17) appartenant à ladite commande électronique extérieure (6); **caractérisée en ce que** les moyens permettant d'accoupler l'axe (8) de la cloche obturante sur l'axe (16) du moteur à engrenage (17) comprennent une douille (18) accouplée sur l'axe (8) de la cloche obturante et munie d'une vis (20) qui est insérée dans son ouverture supérieure (19), ladite vis se vissant dans un filet (21) axial dudit axe (8) et comprenant une tête (22) présentant des entrants (23) aptes à recevoir l'accouplement de saillies (24) de l'axe (16) du moteur à engrenage (17).

2. Valve distributrice pour liquides, d'après la revendication 1, **caractérisée en ce que** la périphérie extérieure de la douille (18) forme une denture (25) de telle façon qu'avec la coopération d'un doigt à ressort (26) qui est prévu dans la base (11) qui sert comme support de la commande électronique extérieure (6), ladite denture constitue un cliquet permettant de stabiliser les différentes positions de rotation de la cloche obturante, ladite douille (18) présentant radialement un nez (28) qui est apte à agir sur les microrupteurs (29) équipant le circuit électronique (30) de la commande (6) à fin d'ainsi déterminer lesdites positions de la cloche obturante.

3. Valve distributrice pour liquides, d'après la revendication 1, **caractérisée en ce que** la vis (20) qui est en accouplement avec l'axe (8) de la cloche obturante présente une collerette (20') qui heurte contre un interrupteur de fin de course qui est prévu chez le moteur à engrenage (17) à fin d'ainsi limiter le mouvement ascendant desdites vis (20) et cloche.

4. Valve distributrice pour liquides, d'après la revendication 1, **caractérisée en ce qu'**après l'enlèvement desdites douille (18) et vis (20), l'axe (8) de la cloche obturante est préparé pour revecoir l'accouplement d'une manette permettant l'actionnement manuel de ladite cloche.
